# EUROPEAN PATENT SPECIFICATION

(11) **EP 3 837 008 B1**
(45) Date of publication and mention of the grant of the patent: **24.09.2025**
(21) Application number: 19750132.3
(22) Date of filing: 15.08.2019
(51) Int. Cl.: A61K 31/166, A61P 1/04, A61P 19/02, A61P 29/00, C07C 235/00

(54) **SUBSTITUTED BENZAMIDES AND THEIR USE IN THERAPY**
SUBSTITUIERTE BENZAMIDE UND DEREN VERWENDUNG IN DER THERAPIE
BENZAMIDES SUBSTITUÉS ET LEUR UTILISATION EN THÉRAPIE

(30) Priority: 15.08.2018 EP 18189195
(43) Date of publication of application: 23.06.2021
(73) Proprietor: Pharmacyl AB, 237 36 Bjärred (SE)
(72) Inventor: BJÖRK, Anders, 237 36 BJÄRRED (SE); HEDLUND, Gunnar, 224 56 LUND (SE)
(74) Representative: Brann AB
(86) International application number: PCT/EP2019/071904
(87) International publication number: WO 2020/035555

(56) References cited:
- WO-A1-2018/037103
- WO-A1-2018/037103
- US-A- 6 100 299
- US-A- 6 100 299
- LINDGREN HANNA ET AL: "N-substituted benzamides inhibit nuclear factor-kappaB and nuclear factor of activated T cells activity while inducing activator protein 1 activity in T lymphocytes", MOLECULAR IMMUNOLOGY, PERGAMON, GB, vol. 38, no. 4, 31 July 2001 (2001-07-31), pages 267 - 277, XP009511211, ISSN: 0161-5890, DOI: 10.1016/S0161-5890(01)00060-8
- LINDGREN HANNA ET AL: "N-substituted benzamides inhibit nuclear factor-kappaB and nuclear factor of activated T cells activity while inducing activator protein 1 activity in T lymphocytes", MOLECULAR IMMUNOLOGY, PERGAMON, GB, vol. 38, no. 4, 31 July 2001 (2001-07-31), pages 267 - 277, XP009511211, ISSN: 0161-5890, DOI: 10.1016/S0161-5890(01)00060-8

## Description

### FIELD OF THE INVENTION

The present invention relates to compounds that are useful in the treatment of certain diseases resulting from pathologic inflammation. More particularly, the invention relates to 4-alkanoylaminobenzamide derivatives and their use in therapy, in particular for the treatment of certain diseases resulting from pathologic inflammation. Furthermore, the invention relates to pharmaceutical formulations comprising such compounds.

### BACKGROUND OF THE INVENTION

Throughout this application, various (non-patent) publications are referred to by first author and year of publication. Full citations for these publications are presented in a References section immediately before the claims. The disclosures of these documents and publications referred to herein are hereby incorporated in their entireties by reference into this application in order to more fully describe the state of the art to which this invention pertains.

Diseases resulting from pathologic inflammation may be treated by various anti-inflammatory agents, such as corticosteroids, immunomodulators, immunosuppressants, and tumor necrosis factor (TNF) inhibitors. TNF is involved in various inflammatory, autoimmune and immune-mediated disorders, e.g. inflammatory bowel disease (IBD), rheumatoid arthritis (RA), and psoriasis and this forms the basis for the use of TNF inhibitors (anti-TNF drugs) in the treatment of such diseases. However, there are some important side effects linked to the anti-TNF drugs including increased risk of lymphoma and skin cancer, increased risk for severe infections, including tuberculosis and fungal infections, and injection site reactions.

Not all patients treated with anti-TNF drugs respond to such therapy. For example, among IBD patients treated with current biologic anti-TNF drugs, only approximately 20% to 35% of patients will achieve full remission (Sandborn, 2016). In addition, patients who do not experience full remission, lose response over time, or have no response to anti-TNF therapy, have a substantially decreased response rate also when treated with a second anti-TNF drug (Sands, 2014). Moreover, while the anti-TNF drugs may have a rapid onset of action with regard to symptomatic improvement in IBD patients, only a moderate correlation exists between this improvement and reduction in mucosal inflammation. The rapid control of symptoms is likely due to direct neutralisation of circulating TNF, but other mechanisms of action are required to decrease the total inflammatory burden and induce mucosal healing (Hindryckx, 2018).

Many diseases resulting from pathologic inflammation are of chronic nature, or at least may require long term treatment. In such cases, it is of utmost importance to try to reduce as far as possible any serious side effects resulting from the treatment. For example, while the 4-aminobenzamide derivatives such as procainamide (CAS No. 614-39-1) and declopramide (CAS No. 891-60-1) were suggested as potential therapeutic agents for the treatment of IBD (Kim, 2016; Ivanenkov, 2011), well-known neurological and cardiovascular side effects linked to this chemical class of compounds may outweigh potential clinical benefits in long-term treatment (Harrington, 1983; Harron, 1990).

IBD, mainly comprised of Crohn's disease (CD) and ulcerative colitis (UC), is a chronic, frequently progressive condition of the gastrointestinal tract that requires lifelong treatment. The natural history of the condition is one of periods of remission, punctuated by relapses of disease activity. CD and UC are diseases with many similarities and share many overlapping epidemiological, clinical and therapeutic characteristics, but differ frequently with respect to localization and how they are managed.

UC is a disease in which the innermost lining of the colon and rectum becomes inflamed. Histologically, UC is characterised by the presence of both acute and chronic inflammation. The inflammation causes the bowel to empty frequently. Ulcers form, which then bleed and produce pus and mucous. The hallmark symptom of UC is bloody diarrhoea accompanied by urgency to defecate. Other symptoms include crampy abdominal pain, loss of appetite, fatigue, weight loss and fevers. The diagnosis of UC is based on a combination of symptoms, endoscopic findings, and histology. The pathogenesis is multifactorial, involving genetic predisposition, dysregulated immune responses, and environmental factors.

CD typically presents with abdominal pain, diarrhoea, and weight loss. The disease most often begins gradually and may affect only a small part of the gastrointestinal tract, but it has the potential to progress extensively over time. CD can cause inflammation that goes through the wall of the intestines causing fistulas or abscesses. Most commonly, CD affects the small intestine and the beginning of the large intestine. The disease, however, can affect any part of the digestive tract, from the mouth to the anus.

Depending on the extent of disease, UC patients can be classified as having 1) ulcerative proctitis involving only the rectum, 2) left sided UC involving the colorectum distal to the splenic flexure and 3) extensive UC involving the colon proximal to the splenic flexure (includes pancolitis). Symptoms may vary depending on the degree and extent of inflammation. Patients are classified as having mild, moderate or severe disease activity according to one or more measures of disease severity. People are more frequently diagnosed for UC between the ages of 15 and 35, and at a second lesser peak between 55 and 65 years of age. The median age at diagnosis is 30 years. In 15% of cases UC is diagnosed in childhood and may present before school age. Traditionally, the highest occurrence of both CD and UC is found in the developed countries of North America and Europe. The prevalence is estimated to be 70-500 cases per 100.000, with males and females affected equally. CD and UC account for substantial costs to the health care system and society.

UC has a substantial impact on the patient's quality of life due to early onset and lack of a cure. Current medications are moderately efficacious, and adverse effects are a problem. It is therefore urgent to improve clinical management by establishing new treatment strategies. The therapeutic goals are induction and maintenance of remission and prevention of complications. Patients with mucosal healing who have no or very mild symptoms and signs are considered in remission. Effect on the inflammatory process is essential since lack of control of inflammation even in the presence of control of symptoms is correlated with poor long-term outcome with a higher risk of early relapse and/or poor long-term outcome including raised risk of colectomy and colorectal cancer. Extra-intestinal manifestations of UC include primary sclerosing cholangitis, as well as joint, skin and eye manifestations.

Historically, UC drugs were developed primarily for symptom-based treatment, i.e., induction and maintenance of symptomatic improvement, or at best clinical remission, and prevention of complications, without significantly altering the natural course of the disease. In the recent decades, treatment goals in both UC and CD patients have evolved greatly from focusing on just clinical response to the need to achieve better outcomes associated with healing of mucosal lesions. Mucosal healing (MH) has become a key step to achieve sustained remission in UC (Frøslie 2007; Peyrin-Biroulet 2015), and nowadays the regulatory agencies are requiring that treatments not only show an improvement in the signs and symptoms of the disease, but also an improvement in endoscopic disease activity and, ideally, healing of the bowel mucosa (EMA 2016). The implementation of these recommendations in clinical practice has the potential to change disease course and to restore the patient's quality of life.

Current medications are moderately efficacious, and adverse effects are a problem. Therapeutic recommendations depend on the disease location, disease severity, and disease complications. For example, first-line therapy in mild to moderate UC is the 5-aminosalicylate (5-ASA) agents, i.e. mesalazine, sulfasalazine, olsalazine, and balsalazide. Sulfasalazine (SASP) is the parent 5-ASA agent. For the approximately 50% of UC patients who fail 5-ASA therapy, the next line of treatment is corticosteroids and/or immunomodulators (thiopurines). SASP provides a modest benefit for the treatment of mild to moderately active CD (Lim, 2016).

Biologic drugs, e.g., anti-TNF drugs, are generally indicated for the treatment of patients who fail the conventional therapies. Drawbacks and shortcomings of such drugs have been discussed herein above.

There is presently no cure for IBD, and the goal of treatment is to reduce symptoms, achieve and maintain remission and avoid complications as far as possible. Thus, medication may be anti-inflammatory, immunomodulating, immunosuppressant, anti-diarrheal etc. to combat the various symptoms of the disease.

Some 4-alkanoylaminobenzamides have been previously described, with or without indication of a particular use.

Thus, US Patent Application No. 2011/0027179 discloses 4-acetamido-N-[2-(diethylamino)ethyl]-2-methoxybenzamide, N-[2-(diethylamino)ethyl]-2-methoxy-4-(propanoylamino)benzamide, N-[2-[di(propan-2-yl)amino]ethyl]-2-ethoxy-4-(propanoylamino)benzamide, N-[2-(diethylamino)ethyl]-2-ethoxy-4-(propanoylamino)benzamide, 4-propionylamino-2-methoxy-N-(2-morpholin-4-yl-ethyl)-benzamide, 4-propionylamino-N-(2-diisopropylamino-ethyl)-2-ethoxy-benzamide, 4-propionylamino-N-(2-dibutylamino-ethyl)-2-ethoxy-benzamide, 4-propionylamino-2-methoxy-N-(2-piperidin-1-yl-ethyl)-benzamide, and 4-propionylamino-2-methoxy-N-(2-pyrrolidin-1-yl-ethyl)-benzamide. US Patent Application No. 2011/0027179, however, is directed to radiohalogenated benzamide derivatives and their use in tumor diagnosis and tumor therapy, which means that the disclosed compounds supposedly are used as synthesis intermediates.

U.S. Patent No. 3,177,252 discloses compounds of a generic formula that includes 4-alkanoylaminobenzamide, for the treatment of emesis, and behaviour disturbances, without however mentioning any particular example of a 4-alkanoylaminobenzamide.

WO 2014/064229 discloses the use the 4-alkanoylaminobenzamides to enhance the protective immunity elicited by an immunogen. Mention is made of 4-acetamido-3-chloro-N-[2-(diethylamino)ethyl]benzamide, 4-acetamido-5-chloro-N-[2-(diethylamino)ethyl]-2-methoxybenzamide, and 4-acetamido-N-[2-(diethylamino)ethyl]benzamide.

WO 2005/025498 indicates that some 4-alkanoylaminobenzamides, when delivered topically, cause cellular (eosinophil) apoptosis and, based on this effect, discloses the use of such compounds for the treatment of acute or chronic respiratory tract inflammation associated with eosinophil infiltration. Mention is made of 4-acetamido-N-[2-(diethylamino)ethyl]benzamide and N-[2-(diethylamino)ethyl]-4-(pentanoylamino)benzamide.

WO 99/63987 discloses 4-acetamido-3-chloro-N-[2-(diethylamino)ethyl]benzamide (N-acetyldeclopramide) and uses thereof for inhibiting or killing tumour or cancer cells and as potentially useful in a method of treating inflammatory disorders.

Acecainide (4-acetamido-N-[2-(diethylamino)ethyl]benzamide; CAS No. 32795-44-1) has been used for the treatment or prevention of cardiac arrhythmias.

The compounds 4-acetylamino-N-(2-morpholin-4-yl-ethyl)benzamide and N-(2-morpholin-4-ylethyl)-4-(pentanoylamino)benzamide are commercially available, but no particular use has been suggested.

WO 2018/037103 discloses procainamide for use in the prophylaxis or treatment of an immune related disease, disorder or condition, including IBD.

As noted herein above, some patients suffering from inflammatory diseases do not respond to anti-TNF-α treatment, or respond poorly to such treatment, and for such patients other modes of treatment must be provided.

### SUMMARY OF THE INVENTION

A primary objective of the present invention is to provide a compound for use in a new method for the treatment of IBD with the final aim of changing the disease course. The present inventors contemplate that a more effective treatment of IBD than today's may necessitate drugs that affect not just one transcription factor (TF), like NFκB, but that simultaneously affect two or more TFs. Combinatorial regulation is a powerful mechanism that enables tight control of gene expression via integration of multiple signaling pathways, inducing different TFs required for promoting the initiation of very specific biological responses, which may involve functional synergy or cooperative interactions between two or more different TFs. Without wishing to be bound to any theory, the present inventors have found that compounds of formula (I) are exceptionally efficacious in reducing symptoms of IBD and contemplate that such superior efficacity may be based on a multiple effect of the compounds in the cell of a mammal, viz. inhibition of NFκB and NFAT, and enhancement of AP-1 activity. Such combined activity may be the reason for the very advantageous effect obtained on e.g. the intestinal mucosa, with astonishingly high mucosal healing, as observed by cessation of rectal bleeding, a reliable symptom score for identifying the level of mucosal inflammation.

Thus, a first aspect is a compound of formula (I) 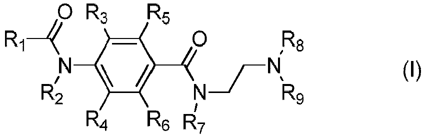 or a pharmaceutically acceptable salt or solvate thereof; wherein
R₁ is selected from C1-C6 alkyl and C3-C6 cycloalkyl;
R₂ is selected from H and C1-C3 alkyl;
R₃, R₄, R₅, and R₆ are independently selected from hydrogen, C1-C3 alkyl, C1-C3 alkoxy, C1-C3 alkylthio, fluoro, chloro, and bromo, wherein any alkyl is optionally substituted with one or more fluoro;
R₇ is selected from hydrogen and C1-C3 alkyl; and
R₈ and R₉ are independently selected from C1-C6 alkyl;
for use in the treatment of an inflammatory bowel disease.

A further aspect is a compound selected from
4-propanoylamino-3-chloro-N-[2-(diethylamino)ethyl]benzamide, and
4-isobutyrylamino-3-chloro-N-[2-(diethylamino)ethyl]benzamide, or
a pharmaceutically acceptable salt or solvate thereof.

A further aspect is a compound selected from
4-propanoylamino-3-chloro-N-[2-(diethylamino)ethyl]benzamide, and
4-isobutyrylamino-3-chloro-N-[2-(diethylamino)ethyl]benzamide, or
a pharmaceutically acceptable salt or solvate thereof, for use in therapy.

In some embodiments, a pharmaceutical formulation is provided, comprising a compound selected from
4-propanoylamino-3-chloro-N-[2-(diethylamino)ethyl]benzamide, and
4-isobutyrylamino-3-chloro-N-[2-(diethylamino)ethyl]benzamide,
or a pharmaceutically acceptable salt or solvate thereof, and optionally a pharmaceutically acceptable excipient.

One advantageous aspect is a compound of formula (I) as defined herein, for use in the treatment of an inflammatory bowel disease in a mammal for which conventional anti-TNF-α treatment (using TNF-α antagonists) is not useful, e.g. for use in the treatment of a patient for which conventional anti-TNF-α treatment has failed or is contraindicated.

In some embodiments, a compound of formula (I) as defined herein above is provided for use in the treatment of at least one symptom of IBD, e.g. at least one symptom selected from diarrhoea, rectal bleeding, reduced body weight, mucosal ulceration, intestinal crypt destruction and infiltration of leukocytes into intestinal mucosa.

In some embodiments, a compound of formula (I) is provided for use in the treatment of mucosal ulceration in connection with IBD.

In some embodiments, a compound of formula (I) is provided for use in the treatment of refractory lesions of the intestinal mucosa in IBD.

One further aspect is a pharmaceutical composition comprising a combination of a compound of formula (I) as defined herein, and a further therapeutically active ingredient, and optionally a pharmaceutically acceptable excipient, for the treatment of IBD.

Other features and advantages of the invention will be understood by references to the detailed description and examples that follow.

### DETAILED DESCRIPTION OF THE INVENTION

### Definitions

As used herein, and unless stated otherwise and unless otherwise apparent from the context, each of the following terms shall have the definition set forth below.

The term "Cn alkyl" refers to a linear or branched chain saturated hydrocarbyl radical containing n carbon atoms in the chain, i.e. a moiety of formula CₙH₂ₙ₊₁.

The term "Cn-Cm alkyl" refers to a linear or branched chain alkyl radical containing a number of carbon atoms in the chain ranging from n to m, wherein n and m are both integers and m is higher than n.

The term "Cn cycloalkyl" refers to a cyclic hydrocarbyl radical of formula CₙH₂ₙ₋₁.

The term "Cn-Cm cycloalkyl" refers to a cyclic hydrocarbyl radical containing a number of carbon atoms in the cycle ranging from n to m, wherein n and m are both integers and m is higher than n.

The term "Cn-Cm alkoxy" refers to a moiety of formula 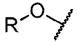 wherein R is Cn-Cm alkyl. For example, methoxy is C1 alkoxy.

The term "Cn-Cm alkylthio" refers to a moiety of formula 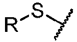 wherein R is Cn-Cm alkyl. For example, methylthio is C1 alkylthio.

The term "halogen" refers to F, Cl, Br or I; preferably F, Cl or Br.

The term "hydroxy" refers to the moiety

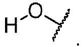

The term "phenyl" refers to a radical of formula

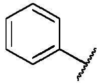

As used herein, "AABZ" means a compound of formula (I) as described herein, unless otherwise indicated or apparent from the context. Furthermore, unless otherwise indicated or apparent from the context, the term also includes a pharmaceutically acceptable salt or solvate (including hydrate) thereof.

As used herein, "about" in the context of a numerical value or range means ±20% of the numerical value or range recited or claimed.

As used herein, "administration", "administering" etc. means the giving of, dispensing of, or application of medicines, drugs, or remedies to a subject (e.g. a mammal subject, preferably a human) to relieve or cure a pathological condition. Oral administration is one way of administering the instant compounds to the subject.

As used herein, an "amount" or "dose" of a compound (e.g. AABZ) as measured in milligrams refers to the milligrams of the compound present in a preparation, regardless of the form of the preparation. A "dose of 5.0 mg of a compound" means the amount of compound in a preparation is 5.0 mg, regardless of the form of the preparation. Thus, when in the form of a salt, e.g. a hydrochloride, the weight of the salt form necessary to provide a dose of 5.0 mg of the free base compound would be greater than 5.0 mg due to the presence of the additional acid.

As used herein, "anti-TNF-α treatment" refers to treatment aiming to reduce TNF-α activity and/or TNF-α production, normally for a therapeutic purpose, such as for the treatment of an inflammatory disorder.

As used herein, "AP-1" means activator protein-1.

As used herein, "combination" means an assemblage of compounds for use in therapy either by simultaneous or separate (e.g. sequential or concomitant) administration. Simultaneous administration refers to administration of an admixture (whether a true mixture, a suspension, an emulsion or other physical combination) of two active ingredients (e.g. the AABZ and a further, therapeutically active agent). In this case, the combination may be an admixture of the AABZ and the further agent; or the AABZ and the further agent may be provided in separate containers and combined just prior to administration. Separate administration may be sequential (i.e. consecutive) or concomitant (i.e. happening at the same time).

Separate administration refers to the concomitant or sequential administration of the AABZ and the further therapeutically active agent as separate formulations, but at the same time or at times sufficiently close together for an activity to be observed that is at least additive, relative to the activity of either one of the AABZ and the further, therapeutically active agent alone.

As used herein, "CD" means Crohn's disease.

As used herein, "Cpd A" means 4-acetamido-3-chloro-N-[2-(diethylamino)ethyl]benzamide (N-acetyldeclopramide) hydrochloride.

As used herein, "DSS" means Dextran Sulfate Sodium.

As used herein, "effective" when referring to an amount of a therapeutically active agent, e.g. AABZ, refers to the amount that is sufficient to yield a desired therapeutic response without undue adverse side effects (such as toxicity, irritation, or allergic response), commensurate with a reasonable benefit/risk ratio when used in the manner of this invention.

As used herein, "excipient" refers to a substance formulated alongside the active ingredient of a medication included for such purposes as long-term stabilization, to provide bulk to a solid formulation, to act as a carrier and/or diluent, to confer a therapeutic enhancement on the active ingredient in the final dosage form, e.g. by facilitating absorption, reducing viscosity, or enhancing solubility. An excipient can also be useful in the manufacturing process, e.g. by facilitating powder flowability or providing non-stick properties. Examples of excipients are antiadherents, binders, coatings, colours, disintegrants, flavours, glidants, lubricants, preservatives, sorbents, sweeteners, and vehicles (carriers).

As used herein "IκB" means inhibitor of kappa B.

As used herein "inflammatory bowel disease" or "IBD" refers to diseases of the bowel associated with inflammation and/or ulceration and includes e.g. Crohn's disease and ulcerative colitis.

As used herein, "inhibition" of disease progression or disease complication in a subject means preventing or reducing the disease progression and/or disease complication in the subject.

As used herein, "MH" means mucosal healing.

As used herein, "mucosal healing" refers to the reduction of mucosal ulceration, e.g. of the intestinal mucosa. The healing may be complete or partial, and may be permanent or not, e.g. persisting over a time period of several weeks, months or years. The mucosal healing may be observed by lessening of symptoms such as diarrhoea, rectal bleeding and pain. Mucosal healing may also be ascertained by endoscopic examination (colonoscopy).

As used herein, "NFAT" means nuclear factor of activated T cell.

As used herein, "NFκB" means nuclear factor kappa B.

As used herein, "patient" or "subject" refers to a mammal patient or subject, selected from animals and humans.

As used herein, "pharmaceutically acceptable" refers to that which is suitable for use with humans and/or animals, generally safe and non-toxic at normal use, i.e. without undue adverse side effects (such as toxicity, irritation, and allergic response) commensurate with a reasonable benefit/risk ratio.

As used herein, "SASP" means sulfasalazine.

As used herein, a "salt thereof" is a salt of the instant compounds which have been modified by making acid or base salts of the compounds. The term "pharmaceutically acceptable salt" in this respect, refers to the relatively non-toxic, inorganic and organic acid or base addition salts of compounds of the present invention. For example, one means of preparing such a salt is by treating a compound of the present invention with an inorganic acid.

As used herein, a "solvate" means a physical association of a compound (e.g. a compound of formula (I)) with one or more solvent molecules, e.g. by hydrogen bonding. "Solvate" encompasses both solution-phase and isolable solvates. Exemplary solvates include, but are not limited to, hydrates, ethanolates, methanolates, and isopropanolates. Methods of solvation are generally known to the person of ordinary skill in the art.

As used herein, a "symptom" associated with a disease resulting from pathologic inflammation includes any clinical or laboratory manifestation associated with the disease and is not limited to what the subject can feel or observe.

As used herein, "TF" means transcription factor, a protein that controls the rate of transcription of genetic information from DNA to messenger RNA.

As used herein, "TNF" means tumour necrosis factor.

As used herein, "TNF-α" means tumour necrosis factor alpha.

As used herein, "treating" encompasses, e.g., inducing inhibition, regression, or stasis of a disease or disorder, e.g., IBD, or alleviating, lessening, suppressing, inhibiting, reducing the severity of, eliminating or substantially eliminating, or ameliorating a symptom of the disease or disorder.

As used herein, "UC" means ulcerative colitis.

### The compound of formula (I)

In a compound of formula (I) as defined herein, R₁ is selected from C1-C6 alkyl and C3-C6 cycloalkyl. In some embodiments, R₁ is selected from C1-C5 alkyl and C3-C5 cycloalkyl, e.g. from C1-C4 alkyl and C3-C4 cycloalkyl, or from C1-C3 alkyl and C3 cycloalkyl. In some embodiments, R₁ is selected from C1-C6 alkyl, e.g. from C1-C5 alkyl or from C1-C4 alkyl or from C1-C3 alkyl, or from C1-C2 alkyl. In some embodiments, R₁ is selected from methyl, ethyl, n-propyl, iso-propyl, cyclopropyl, n-butyl, sec-butyl, iso-butyl, tert-butyl, cyclobutyl, n-pentyl, sec-pentyl, iso-pentyl, tert-pentyl, neo-pentyl, and cyclopentyl; e.g. from methyl, ethyl, n-propyl, iso-propyl, cyclopropyl, n-butyl, sec-butyl, iso-butyl, tert-butyl, n-pentyl, sec-pentyl, iso-pentyl, tert-pentyl, and neo-pentyl. In some embodiments, R₁ is selected from methyl, ethyl, n-propyl, iso-propyl, cyclopropyl, n-butyl, sec-butyl, iso-butyl, tert-butyl, and cyclobutyl, e.g. from methyl, ethyl, n-propyl, iso-propyl, cyclopropyl, n-butyl, sec-butyl, iso-butyl, and tert-butyl. In some embodiments, R₁ is selected from methyl, ethyl, n-propyl, iso-propyl, and cyclopropyl, e.g. from methyl, ethyl, n-propyl, and iso-propyl. In some embodiments, R₁ is selected from methyl, ethyl, n-propyl, iso-propyl, n-butyl, sec-butyl, iso-butyl, tert-butyl, n-pentyl, sec-pentyl, iso-pentyl, tert-pentyl, and neo-pentyl; e.g. from methyl, ethyl, n-propyl, iso-propyl, n-butyl, sec-butyl, iso-butyl, and tert-butyl; or from methyl, ethyl, n-propyl, and iso-propyl; e.g. from methyl, ethyl, and iso-propyl. In some embodiments, R₁ is methyl or ethyl. In some embodiments, R₁ is methyl. In some embodiments, R₁ is ethyl. In some embodiments, R₁ is iso-propyl.

The moiety R₂ is selected from hydrogen and C1-C3 alkyl. In some embodiments, R₂ is selected from hydrogen, methyl, ethyl, n-propyl and iso-propyl; e.g. from hydrogen, methyl and ethyl, or from hydrogen and methyl. In some embodiments, R₂ is hydrogen.

The moieties R₃, R₄, R₅, and R₆ are independently selected from hydrogen, C1-C3 alkyl, C1-C3 alkoxy, C1-C3 alkylthio, fluoro, chloro, and bromo, wherein any alkyl is optionally substituted with one or more fluoro. In some embodiments, R₃, R₄, R₅, and R₆ are independently selected from hydrogen, C1-C3 alkyl, C1-C3 alkoxy, fluoro, chloro, and bromo, wherein any alkyl is optionally substituted with one or more fluoro. In still other embodiments, R₃, R₄, R₅, and R₆ are independently selected from hydrogen, C1-C3 alkyl, fluoro, chloro, and bromo, wherein any alkyl is optionally substituted with one or more fluoro. In still other embodiments, R₃, R₄, R₅, and R₆ are independently selected from hydrogen, C1-C3 alkoxy, fluoro, chloro, and bromo, wherein any alkyl is optionally substituted with one or more fluoro. In still other embodiments, R₃, R₄, R₅, and R₆ are independently selected from hydrogen, C1-C3 alkoxy, C1-C3 alkylthio, fluoro, chloro, and bromo, wherein any alkyl is optionally substituted with one or more fluoro. In still further embodiments, R₃, R₄, R₅, and R₆ are independently selected from hydrogen and fluoro, chloro, bromo.

When any one of R₃, R₄, R₅, and R₆ is selected from C1-C3 alkyl, it e.g. may be selected from methyl and ethyl; in particular it may be methyl.

When any one of R₃, R₄, R₅, and R₆ is selected from C1-C3 alkoxy, it e.g. may be selected from methoxy and ethoxy; in particular it may be methoxy. In some embodiments, R₅ and R₆ are not C1-C3 alkoxy.

When any one of R₃, R₄, R₅, and R₆ is selected from C1-C3 alkylthio, it e.g. may be selected from methylthio and ethylthio; in particular it may be methylthio.

In some embodiments, when any one of R₃, R₄, R₅, and R₆ is a halogen selected from fluoro, chloro and bromo, said halogen is selected from chloro and bromo, in particular it is chloro. In some further embodiments, when any one of R₃, R₄, R₅, and R₆ is a halogen selected from fluoro, chloro and bromo, said halogen is selected from fluoro and chloro.

In some embodiments, R₃, R₄, R₅, and R₆ are independently selected from hydrogen, methyl, ethyl, methoxy, ethoxy, methylthio, fluoro, chloro, bromo, and trifluoromethyl; or from hydrogen, methyl, ethyl, methoxy, ethoxy, fluoro, chloro, bromo, and trifluoromethyl; e.g. from hydrogen, methyl, methoxy, fluoro, chloro, bromo, and trifluoromethyl; or from hydrogen, methyl, methoxy, chloro, and trifluoromethyl, or from hydrogen, methyl, methoxy and chloro; or from hydrogen, methoxy and chloro; or from hydrogen, methyl and chloro; e.g. from hydrogen and chloro.

In some of the above embodiments, at least one, more preferably at least two of R₃, R₄, R₅, and R₆ are hydrogen. In some of the above embodiments, two of R₃, R₄, R₅, and R₆ are hydrogen. In some of the above embodiments, three of R₃, R₄, R₅, and R₆ are hydrogen. In some of the above embodiments, each one of R₃, R₄, R₅, and R₆ is hydrogen. In some of the above embodiments, at least one of R₃, R₄, R₅, and R₆ is different from hydrogen. In some of the above embodiments, one of R₃, R₄, R₅, and R₆ is different from hydrogen and the three others are hydrogen; e.g. one of R₃, R₄, R₅, and R₆ (e.g. R₃) is halogen, such as chloro; and the three others are hydrogen. In some of the above embodiments, R₃ is different from hydrogen, and each one of R₄, R₅, and R₆ is hydrogen. In some embodiments, R₅ is different from hydrogen, and each one of R₃, R₄, and R₆ is hydrogen. In some embodiments, one of R₃, R₄, R₅, and R₆ (e.g. R₃) is fluoro, chloro, or bromo, in particular chloro; and the three others are hydrogen. In some embodiments, R₃ is hydrogen or halogen, e.g. hydrogen or chloro; and R₄, R₅, and R₆ are hydrogen. In some embodiments, R₄, and R₆ are hydrogen; e.g. R₄ and R₆ are hydrogen and at least one of R₃ and R₅ is different from hydrogen, e.g. both R₃ and R₅ are different from hydrogen. In some embodiments, R₄ and R₅ are hydrogen; e.g. R₄ and R₅ are hydrogen and at least one of R₃ and R₆ is different from hydrogen, e.g. both R₃ and R₆ are different from hydrogen. In some embodiments, R₃ and R₄ are hydrogen; e.g. R₃ and R₄ are hydrogen and at least one of R₅ and R₆ is different from hydrogen, e.g. both R₅ and R₆ are different from hydrogen. In some of the above embodiments, R₅ and R₆ are not methoxy or ethoxy.

The moiety R₇ is selected from hydrogen and C1-C3 alkyl. In some embodiments, R₇ is selected from hydrogen, methyl, ethyl, propyl and iso-propyl; e.g. from hydrogen, methyl, ethyl, and propyl. In some embodiments, R₇ is selected from hydrogen, methyl, and ethyl. In some embodiments, R₇ is selected from hydrogen and methyl. In some embodiments, R₇ is hydrogen.

The moieties R₈ and R₉ are independently selected from C1-C6 alkyl. R₈ and R₉ for example may be selected from C1-C5 alkyl; or from C1-C4 alkyl; or from C1-C3 alkyl; e.g. from methyl and ethyl. In some embodiments, both R₈ and R₉ are ethyl.

In some embodiments, R₁ is C1-C6 alkyl; R₂ is hydrogen; R₇ is hydrogen; and R₈ and R₉ are independently selected from C1-C6 alkyl. In some of these embodiments, R₁, R₈ and R₉ are independently selected from C1-C3 alkyl.

In some further embodiments, R₁ is C1-C3 alkyl; R₂, R₄, R₅ and R₇ are hydrogen; and R₈ and R₉ are independently selected from C1-C3 alkyl. In some of these embodiments, R₁ is selected from methyl, ethyl and iso-propyl; and R₈ and R₉ are the same or different and selected from methyl, ethyl and n-propyl. In some further embodiments, R₁ is selected from methyl, ethyl and iso-propyl; R₂, R₄, R₅, R₆, and R₇ are hydrogen; and R₈ and R₉ are the same or different and selected from methyl, ethyl and n-propyl.

In some embodiments, R₂ is selected from hydrogen and methyl; R₃, R₄, R₅, and R₆ are independently selected from hydrogen and chloro; R₇ is hydrogen; and R₈ and R₉ are the same or different and selected from methyl, ethyl and n-propyl.

In some embodiments, R₁ is selected from methyl, ethyl and iso-propyl; R₂ is hydrogen or methyl; R₃ is chloro or hydrogen; R₄, R₅, R₆, and R₇ are hydrogen; and R₈ and R₉ are the same or different and are selected from methyl, ethyl and n-propyl. In some embodiments, R₁ is selected from methyl, ethyl and iso-propyl; R₂ is hydrogen or methyl; R₃ is chloro or hydrogen; R₄, R₅, R₆, and R₇ are hydrogen; and R₈ and R₉ are ethyl. In some embodiments, R₁ is selected from methyl, ethyl and iso-propyl; R₂ is hydrogen; R₃ is chloro or hydrogen; R₄, R₅, R₆, and R₇ are hydrogen; and R₈ and R₉ are the same or different and are selected from methyl, ethyl and n-propyl. In some embodiments, R₁ is selected from methyl, ethyl and iso-propyl; R₂ is hydrogen; R₃ is chloro; R₄, R₅, R₆, and R₇ are hydrogen; and R₈ and R₉ are the same or different and are selected from methyl, ethyl and n-propyl.

In some embodiments, the compound of formula (I) for use as defined herein, is selected from:
4-acetamido-3-chloro-N-[2-(diethylamino)ethyl]benzamide,
4-propanoylamino-3-chloro-N-[2-(diethylamino)ethyl]benzamide,
4-isobutyrylamino-3-chloro-N-[2-(diethylamino)ethyl]benzamide,
4-acetamido-N-[2-(diethylamino)ethyl]benzamide,
4-propanoylamino-N-[2-(diethylamino)ethyl]benzamide, and
4-isobutyrylamino-N-[2-(diethylamino)ethyl]benzamide.

In some embodiments, the compound of formula (I) for use as defined herein, is selected from:
4-acetamido-3-chloro-N-[2-(diethylamino)ethyl]benzamide,
4-propanoylamino-3-chloro-N-[2-(diethylamino)ethyl]benzamide,
4-isobutyrylamino-3-chloro-N-[2-(diethylamino)ethyl]benzamide, and
4-acetamido-N-[2-(diethylamino)ethyl]benzamide.

In some embodiments, the compound of formula (I), for use as defined herein, is selected from:
4-acetamido-3-chloro-N-[2-(diethylamino)ethyl]benzamide,
4-propanoylamino-3-chloro-N-[2-(diethylamino)ethyl]benzamide, and
4-isobutyrylamino-3-chloro-N-[2-(diethylamino)ethyl]benzamide.

In some embodiments, the compound of formula (I), for use as defined herein is 4-acetamido-3-chloro-N-[2-(diethylamino)ethyl]benzamide.

In some further embodiments, a compound of formula (I) for use in therapy is provided, selected from:
4-propanoylamino-3-chloro-N-[2-(diethylamino)ethyl]benzamide, and
4-isobutyrylamino-3-chloro-N-[2-(diethylamino)ethyl]benzamide.

In some embodiments, a compound of formula (I) as disclosed herein is provided, selected from:
4-propanoylamino-3-chloro-N-[2-(diethylamino)ethyl]benzamide, and
4-isobutyrylamino-3-chloro-N-[2-(diethylamino)ethyl]benzamide.

In some embodiments, the compound of formula (I) is 4-propanoylamino-3-chloro-N-[2-(diethylamino)ethyl]benzamide. In some embodiments, the compound of formula (I) is 4-isobutyrylamino-3-chloro-N-[2-(diethylamino)ethyl]benzamide. In some embodiments, the AABZ is a hydrochloride of a compound of formula (I), e.g. 4-acetamido-3-chloro-N-[2-(diethylamino)ethyl]benzamide hydrochloride.

Structural formulas of some compounds mentioned herein are shown in Table 1.

**Table 1**

| | |
|---|---|
| 4-acetamido-3-chloro-N-[2-(diethylamino)ethyl]benzamide | 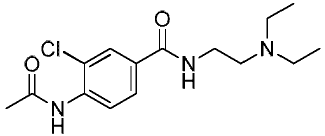 |
| 4-propanoylamino-3-chloro-N-[2-(diethylamino)ethyl]benzamide | 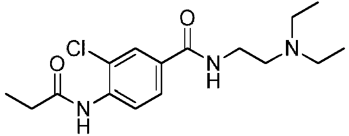 |
| 4-isobutyrylamino-3-chloro-N-[2-(diethylamino)ethyl]benzamide | 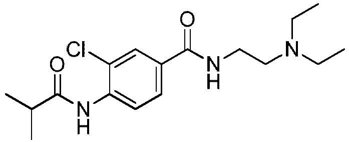 |
| 4-acetamido-N-[2-(diethylamino)ethyl]benzamide | 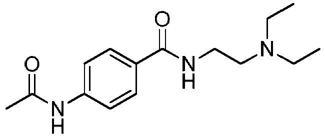 |
| 4-propanoylamino-N-[2-(diethylamino)ethyl]benzamide | 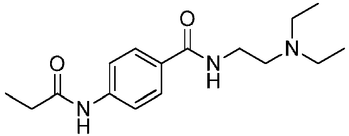 |
| 4-isobutyrylamino-N-[2-(diethylamino)ethyl]benzamide | 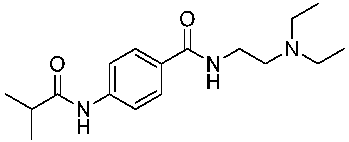 |

### The pharmaceutically acceptable salts

The AABZ may be provided as a pharmaceutically acceptable salt of a compound of formula (I), e.g. an acid addition salt. In the preparation of acid or base addition salts, preferably such acids or bases are used which form suitably therapeutically acceptable salts. Examples of such acids are hydrohalogen acids, sulfuric acid, phosphoric acid, nitric acid, aliphatic, alicyclic, aromatic or heterocyclic carboxylic or sulfonic acids, such as formic acid, acetic acid, propionic acid, succinic acid, glycolic acid, lactic acid, malic acid, tartaric acid, citric acid, ascorbic acid, maleic acid, hydroxymaleic acid, pyruvic acid, p-hydroxybenzoic acid, embonic acid, methanesulfonic acid, ethanesulfonic acid, hydroxyethanesulfonic acid, halogenbenzenesulfonic acid, toluenesulfonic acid or naphthalenesulfonic acid. Base addition salts include those derived from inorganic bases, such as ammonium or alkali or alkaline earth metal hydroxides, carbonates, bicarbonates, and the like, and organic bases such as alkoxides, alkyl amides, alkyl and aryl amines, and the like. Examples of bases useful in preparing salts of the present invention include sodium hydroxide, potassium hydroxide, ammonium hydroxide, potassium carbonate, and the like. In some embodiments the compound of formula (I) is provided as a hydrochloride.

Relevant teachings relating to salt formulations as used herein and processes for preparing the same are described, e.g., in U.S. Pat. No. 3,177,252.

### The pharmaceutically acceptable solvate

Any pharmaceutically acceptable solvate is contemplated as possible according to the present invention. Exemplary solvates include, but are not limited to, hydrates, ethanolates, methanolates, and isopropanolates. In some embodiments, the solvate is a hydrate.

### Methods of preparing the compound of formula (I)

The compounds of formula (I) may be prepared by the person of ordinary skill in the art, e.g. by following the general procedures of US Pat. Appl. No. 2011/0027179, and WO 2005/025498.

For example, a compound of formula (I) may be prepared by a method comprising two consecutive nucleophilic substitution reactions as represented by the following reaction scheme:

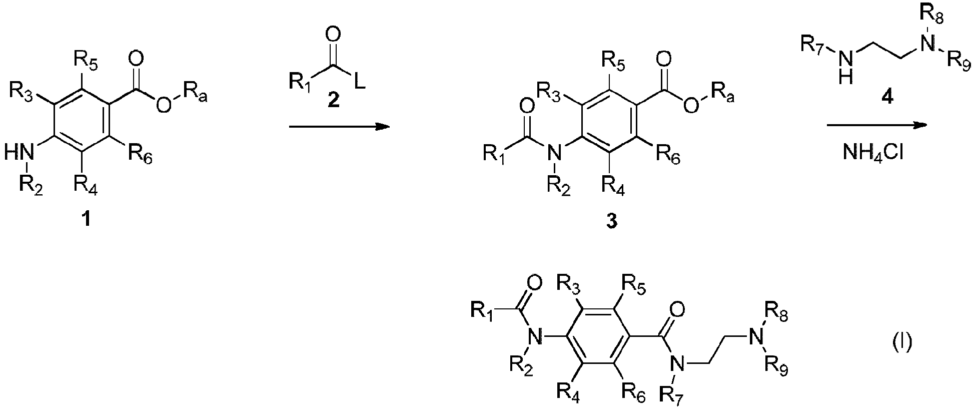

In the above reaction scheme, compound **1,** where Rₐ is e.g. C1-C3 alkyl, is reacted with compound **2,** where L is a suitable leaving group, e.g. Cl, to obtain compound 3. Compound **3** is subsequently reacted with the secondary amine **4** in the presence of ammonium chloride as a catalyst for the reaction, to obtain the compound of formula (I) as defined herein. The compound of formula (I) may optionally be transformed to a suitable pharmaceutically acceptable salt or solvate, e.g. a hydrohalide salt.

### The use of the compound of formula (I)

The compound of formula (I) or the pharmaceutically acceptable salt or solvate thereof (collectively referred to herein below as AABZ) is useful for the treatment of IBD.

In some embodiments the IBD is UC. In some embodiments, the IBD is CD. In some embodiments, the disease is UC or CD.

Very advantageously, the AABZ may be used in the treatment of IBD in a patient for which anti-TNF-α treatment is not considered useful, e.g. because the patient does not provide an adequate response to such treatment or because such treatment for some other reason is contraindicated. In some embodiments, thus, the AABZ is provided for use in the treatment of a patient for which anti-TNF-α treatment has failed or is contraindicated. For example, the patient may be one suffering from an IBD, and for which anti-TNF-α treatment (i.e. treatment using TNF-α antagonists) does not provide adequate relief from symptoms of the IBD, or which for some other reason may not be treated by such approach.

Symptoms vary depending on the severity of inflammation and may range from mild to severe. Signs and symptoms that are common to both CD and UC include diarrhoea, fever and fatigue, abdominal pain and cramping, blood in the stool and reduced appetite.

One particularly advantageous embodiment is the AABZ for use in the treatment of a patient suffering from IBD, e.g. UC or CD, for which anti-TNF-α treatment has failed or is contraindicated. A further advantageous aspect is the AABZ for use in the treatment of mucosal ulcerations, e.g. mucosal ulcerations of the intestine, in connection with IBD.

An advantageous feature of the AABZ is linked to its low affinity for the dopamine D2 receptor, which substantially reduces the risk for the development of adverse movement disorders such as short-term extrapyramidal disorders and tardive dyskinesia. One aspect therefore is an AABZ that may be used in long term treatment of chronic IBD without the negative effects of dopamine D2 receptor blockade.

In some embodiments, the AABZ is a pharmaceutically acceptable salt of a compound of formula (I), in particular a hydrochloride salt.

In some embodiments, the AABZ is administered via oral administration. In some embodiments, the AABZ is administered in a preferably solid unit formulation, which more preferably is a tablet.

In some embodiments, the AABZ is administered daily.

In some embodiments, the amount of the AABZ administered is 0.1-25 mg/kg (mg of drug per kg of body weight of subject) per day (any weight value being based on the non-salt, non-solvate form).

In other embodiments, the administered amount of the AABZ is 0.3-10 mg/kg per day.

In some embodiments, the amount of the AABZ administered is 5.0-2000 mg/day, or 10-1000 mg/day.

In some embodiment, the AABZ is administered once daily. In other embodiments, the AABZ is administered twice daily. In other embodiments, the AABZ is administered three times daily. In yet other embodiments, the AABZ is administered four times daily.

In some embodiments, the administration of the AABZ continues for at least 2 weeks. In other embodiments, the administration of the AABZ continues for 3 months or more. In yet other embodiments, the administration of the AABZ continues for 12 months or more.

### The treated subject

The subject that is treated according to the present invention is a mammal, including a human and a non-human mammal (an animal). Examples, of non-human mammals are primates, domesticated animals, e.g. farm animals, e.g. cattle, sheep, pigs, horses and the like, as well as pet animals, such as dogs and cats, and the like. In preferred embodiments, the subject is a human. In some other embodiments, the subject is a non-human mammal, e.g. a dog or a horse.

### The pharmaceutical composition

One aspect is a pharmaceutical composition comprising an amount of an AABZ, and optionally (but preferably) at least one pharmaceutically acceptable excipient, such as a carrier, for use in treating a subject afflicted with IBD.

The AABZ can be administered in admixture with suitable pharmaceutical diluents, extenders, or carriers, or any other pharmaceutically acceptable excipients, suitably selected with respect to the intended form of administration and as consistent with conventional pharmaceutical practices. A preferred dosage unit will be in a form suitable for oral administration. The AABZ can be administered alone but is generally mixed with a pharmaceutically acceptable carrier, and administered in the form of a tablet or capsule, liposome, or as an agglomerated powder. Examples of suitable solid carriers include lactose, sucrose, gelatine and agar. Capsule or tablets can be easily formulated and can be made easy to swallow or chew; other solid forms include granules, and bulk powders.

Tablets may contain suitable binders, lubricants, disintegrating agents, colouring agents, flavouring agents, flow-inducing agents, and melting agents. For instance, for oral administration in the dosage unit form of a tablet or capsule, the AABZ can be combined with an oral, non-toxic, pharmaceutically acceptable, inert carrier such as lactose, gelatine, agar, starch, sucrose, glucose, methyl cellulose, dicalcium phosphate, calcium sulphate, mannitol, sorbitol, microcrystalline cellulose and the like. Suitable binders include starch, gelatine, natural sugars such as glucose or beta-lactose, corn starch, natural and synthetic gums such as acacia, tragacanth, or sodium alginate, povidone, carboxymethylcellulose, polyethylene glycol, waxes, and the like. Lubricants used in these dosage forms include sodium oleate, sodium stearate, sodium benzoate, sodium acetate, sodium chloride, stearic acid, sodium stearyl fumarate, talc and the like. Disintegrators include, without limitation, starch, methyl cellulose, agar, bentonite, xanthan gum, croscarmellose sodium, sodium starch glycolate and the like.

The amount of AABZ in a dose unit of the formulation (e.g. a tablet or capsule) may be e.g. 5-500 mg (any amount is based on the compound of formula (I) in non-salt form and non-solvated form). In some embodiments, the amount of AABZ is 10-100 mg. In other embodiments, the amount of AABZ is 5-25 mg. In one embodiment, the amount of AABZ in the composition is 500 mg. In other embodiments, the amount of AABZ is 25 mg. In other embodiments, the amount of AABZ is 100 mg. In other embodiments, the amount of AABZ is 10 mg. In other embodiments, the amount of AABZ is less than 10 mg.

In some embodiments the total amount of the AABZ is between 0.1 and 95% by weight of the formulation, e.g. between 0.5 and 50% by weight, or between 1 and 20% by weight, the remainder comprising e.g. a carrier and any other excipient.

In some embodiments, the pharmaceutical composition comprises 4-acetamido-3-chloro-N-[2-(diethylamino)ethyl]benzamide or pharmaceutically acceptable salt thereof, such as the hydrochloride salt.

### Combined use of an AABZ and a further therapeutically active ingredient

One aspect herein is a compound of formula (I) as defined herein, or a pharmaceutically acceptable salt or solvate thereof (an AABZ), for use in combination with at least one further therapeutically active ingredient, in the treatment of IBD, in a mammal for which TNF-α inhibition is not useful.

In some embodiments, the treatment comprises administering to a subject (a mammal patient, e.g. a human patient) an amount of an AABZ, and a further therapeutically active ingredient, wherein the amounts when taken together are effective to treat the subject. In some embodiments, the total amount of the AABZ and the further therapeutically active ingredient when administered together to treat a subject produces an overall better effect, i.e., a synergistic effect, than the simple sum of effects produced when either component, at the same total amount, is administered alone.

The further therapeutically active ingredient may be selected e.g. from nonsteroidal antiinflammatory drugs (NSAIDs), corticosteroids, cytotoxic drugs, immunosuppressive drugs and antibodies.

In some embodiments, the treatment comprises administering to a subject (a mammal patient, e.g. a human patient) an AABZ as an add-on therapy or in combination with a further therapeutically active ingredient in treating a subject afflicted with IBD (e.g. UC or CD). Preferably, the treatment provides induction and maintenance of clinical remission in patients with mild to moderate to severe disease activity. In some embodiments, the treatment is intended for a patient in remission in order to prevent disease relapse.

In some embodiments, the further therapeutically active ingredient is administered via oral administration. In yet other embodiments, the further therapeutically active ingredient is administered via rectal administration.

In some embodiments, the further therapeutically active ingredient is administered once daily. In other embodiments, the further therapeutically active ingredient is administered twice daily. In other embodiments, the further therapeutically active ingredient is administered three times daily. In yet other embodiments, the further therapeutically active ingredient is administered four times daily.

The amount of the AABZ and the amount of the further therapeutically active ingredient when taken together preferably are effective to alleviate a symptom of IBD in the subject.

In some embodiments, the administration of the further therapeutically active ingredient substantially precedes the administration of the AABZ, i.e. the subject is receiving therapy by administration of the further therapeutically active ingredient prior to initiating therapy by administration of the AABZ.

In some embodiments, the subject is receiving therapy by administration of the further therapeutically active ingredient for at least 6 months prior to initiating therapy by administration of the AABZ. In some embodiments, the subject is receiving therapy by administration of the further therapeutically active ingredient for at least 12 months prior to initiating therapy by administration of the AABZ. In some embodiments, the subject is receiving therapy by administration of the further therapeutically active ingredient for at least 24 months prior to initiating therapy by administration of the AABZ.

In some embodiments, the administration of the AABZ and the further therapeutically active ingredient continues for at least 2 weeks. In other embodiments, the administration of the AABZ and the further therapeutically active ingredient continues for 3 months or more. In yet other embodiments, the administration of the AABZ and the further therapeutically active ingredient continues for 12 months or more.

In some embodiments, the AABZ and the further therapeutically active ingredient are administered in combination with each other (simultaneously, or separately and concomitantly or sequentially) at the molar ratio of the AABZ to the further therapeutically active ranging from 1:100 to 100:1, from 1:50 to 50:1, from 1:20 to 20:1, from 1:10 to 10:1, from 1:5 to 5:1, or from 1:2 to 2:1, e.g. about 1:1.

In some embodiments, 4-acetamido-3-chloro-N-[2-(diethylamino)ethyl]benzamide or pharmaceutically acceptable salt thereof, such as the hydrochloride salt, and a further therapeutically ingredient are administered in combination with each other.

A further aspect is an AABZ for use as an add-on therapy or in combination with a further therapeutically active ingredient in treating a subject afflicted with IBD.

One aspect is a pharmaceutical composition comprising an amount of an AABZ, an amount of a further therapeutically active ingredient, and optionally at least one pharmaceutically acceptable excipient, such as a carrier, for use in treating a subject afflicted with IBD.

Generally, a suitable pharmaceutical composition is one as described herein above, in connection with a pharmaceutical formulation of an AABZ, but in addition to the AABZ, the composition also contains a further therapeutically active ingredient.

In some embodiments, the molar ratio of the AABZ to the further therapeutically active in the pharmaceutical composition ranges from 1:100 to 100:1, from 1:50 to 50:1, from 1:20 to 20:1, from 1:10 to 10:1, from 1:5 to 5:1, or from 1:2 to 2:1, e.g. it may be about 1:1.

In some embodiments the total amount of the AABZ and the further therapeutically active ingredient is between 0.1 and 95% by weight of the formulation, e.g. between 0.5 and 50% by weight, or between 1 and 20% by weight, the remainder comprising e.g. a carrier and any other excipient.

In some embodiments, the pharmaceutical composition comprises an AABZ as disclosed herein, e.g. 4-acetamido-3-chloro-N-[2-(diethylamino)ethyl]benzamide or pharmaceutically acceptable salt thereof, such as the hydrochloride salt, and a further therapeutically active ingredient, and optionally a pharmaceutically acceptable excipient.

Specific examples of the techniques, pharmaceutically acceptable carriers and excipients that may be used to formulate oral dosage forms of the present invention are described, e.g., in EP 1 720 531 B1. General techniques and compositions for making dosage forms useful in the present invention are described-in the following references: Modern Pharmaceutics, Chapters 9 and 10 (Banker & Rhodes, Editors, 1979); Pharmaceutical Dosage Forms: Tablets (Lieberman et al., 1981); Ansel, Introduction to Pharmaceutical Dosage Forms 2nd Edition (1976); Remington's Pharmaceutical Sciences, 17th ed. (Mack Publishing Company, Easton, Pa., 1985) (and subsequent editions); Advances in Pharmaceutical Sciences (David Ganderton, Trevor Jones, Eds., 1992); Advances in Pharmaceutical Sciences Vol 7. (David Ganderton, Trevor Jones, James McGinity, Eds., 1995); Aqueous Polymeric Coatings for Pharmaceutical Dosage Forms (Drugs and the Pharmaceutical Sciences, Series 36 (James McGinity, Ed., 1989); Pharmaceutical Particulate Carriers: Therapeutic Applications: Drugs and the Pharmaceutical Sciences, Vol 61 (Alain Rolland, Ed., 1993); Drug Delivery to the Gastrointestinal Tract (Ellis Horwood Books in the Biological Sciences. Series in Pharmaceutical Technology; J. G. Hardy, S. S. Davis, Clive G. Wilson, Eds).; Modern Pharmaceutics Drugs and the Pharmaceutical Sciences, Vol. 40 (Gilbert S. Banker, Christopher T. Rhodes, Eds).

The following examples are intended to illustrate the invention without restricting the scope thereof.

### EXAMPLES

### INTERMEDIARY 1

### Ethyl-4-acetamido-3-chlorobenzoate

Ethyl-4-amino-3-chlorobenzoate (2.0 g, 10 mmol) and 1.4 g of triethylamine were dissolved in 20 mL of dichloromethane. 0.9 g of acetyl chloride in 5 mL of dichloromethane was added drop-wise at 0°C. The reaction mixture was allowed to reach room temperature, stirred for 3 hours, washed with water, and dried. The solvents were evaporated to yield 2.0 g of the title product.

### EXAMPLE 1

### 4-Acetamido-3-chloro-N-[2-(diethylamino)ethyl]benzamide hydrochloride

1.5 g of ethyl-4-acetamido-3-chlorobenzoate (1.5 g, 6.2 mmol) was dissolved in 15 mL of N,N-diethylethylenediamine together with a catalytic amount of ammonium chloride. The reaction mixture was refluxed for 3 hours. Dichloromethane was added and washing 4 times with water removed excess diamine. Drying and evaporation of the solvents yielded the free base of the title compound. The residue was dissolved in ethanol-ether and acidified with ethanolic HCl. The solid that precipitated was collected giving the title compound (1.1 g, 3.1 mmol, 50% yield).

1H NMR (DMSO-d6): δ 1,23 (t, 6H), 2,15 (s, 3H), 3,17-3,23 (6H), 3,65 (q, 2H), 7,88 (d, 1H), 7,94 (s, 1H), 8,06 (s, 1H), 9,05 (t, 1H), 9,68 (s, 1H), 10,42 (s, 1H).

### EXAMPLE 2

### 4-Propanoylamino-3-chloro-N-[2-(diethylamino)ethyl]benzamide hydrochloride

4-Amino-N-[2-(diethylamino)ethyl]benzamide hydrochloride (0.5 g, 1.6 mmol), pyridine (5.0 mL). , and propionic anhydride (5.0 mL) were stirred at 50 °C for 2.5 hours. Then the volatiles were removed using a rotary evaporator. Water (5 mL) was added and evaporated. The residue was freeze dried from water and gave the title compound (0.6 g, 100% yield, HPLC purity 98%).

1H NMR (400 MHz, Methanol-d4) δ 8.07 (d, J = 8.6 Hz, 1H), 8.01 (d, J = 2.0 Hz, 1H), 7.83 (dd, J = 8.6, 2.0 Hz, 1H), 3.76 (t, J = 6.2 Hz, 2H), 3.39 (t, J = 6.2 Hz, 2H), 3.34 (q, J = 7.3 Hz, 4H), 2.52 (q, J = 7.6 Hz, 2H), 1.36 (t, J = 7.3 Hz, 6H), 1.23 (t, J = 7.6 Hz, 3H).

### EXAMPLE 3

### 4-Isobutyrylamino-3-chloro-N-[2-(diethylamino)ethyl]benzamide hydrochloride

The compound 4-isobutyrylamino-3-chloro-N-[2-(diethylamino)ethyl]benzamide hydrochloride was obtained in essentially the same manner, by use of isobutyric anhydride instead of propionic anhydride. Purity by HPLC 99%.

1H NMR (400 MHz, Methanol-d4) δ 8.01 (d, J = 2.2 Hz, 1H), 8.00 (d, J = 8.5 Hz, 1H), 7.83 (dd, J = 8.5, 2.0 Hz, 1H), 3.76 (t, J = 6.2 Hz, 2H), 3.39 (t, J = 6.2 Hz, 2H), 3.34 (q, J = 7.3 Hz, 4H), 2.80 (hept, J = 6.8 Hz, 1H), 1.36 (t, J = 7.3 Hz, 6H), 1.24 (d, J = 6.9 Hz, 6H).

### EXAMPLE 4

### 4-Acetamido-N-[2-(diethylamino)ethyl]benzamide hydrochloride

A suspension of 4-amino-N-[2-(diethylamino)ethyl]benzamide hydrochloride (2.5 g, 9.1 mmol) was stirred in 20 mL of anhydrous pyridine. To the suspension was added 10 mL of acetic anhydride.

The reaction was slightly exothermic. After 1 hour the precipitate was filtered off, washed with ethyl acetate, and dried to afford the title compound (2.83 g, 9.0 mmol, 99% yield). Purity by NMR 98%. 1H NMR (400 MHz, Methanol-d4) δ 7.84 (d, 2H), 7.68 (d, 2H), 3.74 (t, 2H), 3.37 (t, 2H), 3.35 - 3.29 (m, 4H), 2.14 (s, 3H), 1.35 (t, 6H).

### Biological assays

UC is an inflammatory disease characterized by the infiltration of inflammatory cells, such as macrophages, into the lesioned colon. Macrophages serve a critical role in the initiation and propagation of inflammatory responses by releasing proinflammatory mediators, such as TNF-α. Lipopolysaccharide (LPS) is a potent initiator of an inflammatory response. During LPS stimulation, NF-κB signaling is activated to regulate the transcription of numerous genes involved in immunity and inflammation to produce proinflammatory cytokines, such as TNF-α. Suppression of LPS-induced TNF-α production in RAW264.7 macrophages treated with AABZ was investigated by evaluation of the generation of TNF-α.

Both dextran sulphate sodium (DSS) and trinitrobenzene sulfonic acid (TNBS) induced colitis are well-established animal models of mucosal inflammation that have been used for over 2 decades in the study of IBD pathogenesis and preclinical studies for new therapeutics. The DSS-induced colitis model has some advantages when compared to other animal models of colitis (Perse 2012) and was therefore chosen for analysis of treatment effects.

### EXAMPLE 5

### Suppression of LPS-Induced TNF-α Production in Macrophages treated with AABZ

The RAW264.7 macrophage line was maintained in DMEM supplemented with 5% FBS at 37°C in a 5% CO2-humidified air environment. The RAW264.7 cells were seeded in 96-well plates at a density of 1×105/ml and a volume of 200 µl/well. The cells were incubated for 24 h in medium supplemented with 10% FBS and were then pre-incubated with or without the indicated concentrations of test substances for 2 h prior to the addition of LPS (1 µg/ml). The supernatants were subsequently harvested at various time points and production of the proinflammatory cytokine TNF-α in the culture medium was determined using a commercially available enzyme-linked immunosorbent assay (ELISA) kit according to the manufacturer's protocol.

Effects of some AABZs of the invention at 1 mM on LPS-induced TNF-production in RAW264.7 murine macrophage cells are shown in Table 2.

**Table 2. Effects of AABZs at 1 mM on LPS-induced TNF-production in RAW264.7 murine macrophage cells**

| **Compound** | **% Inhibition of TNF-production** |
|---|---|
| LPS-treated control | 0 |
| 4-Acetamido-3-chloro-N-[2-(diethylamino)ethyl]benzamide hydrochloride* | 45 |
| 4-Propanoylamino-3-chloro-N-[2-(diethylamino)ethyl]benzamide hydrochloride | 50 |
| 4-Isobutyrylamino-3-chloro-N-[2-(diethylamino)ethyl]benzamide hydrochloride | 63 |
| 4-Acetamido-N-[2-(diethylamino)ethyl]benzamide hydrochloride | 56 |

| | |
|---|---|
| * Cpd A | |

It was observed that different concentrations (0.3, 1.0, and 3.0 mM) of the compounds significantly reduced the expression levels of TNF in comparison with the LPS-treated control group. These results support the usefulness of compounds as disclosed herein, for the treatment of IBD

### EXAMPLE 6

### Assessment of Efficacy of AABZ in Dextran Sulfate Sodium (DSS) induced Inflammatory Bowel Disease in Mice.

The experiment was performed using 6- to 8-week old female Balb/c mice after a one-week acclimatization period. The mice weighed 18-25 g, were purchased from Taconic (Denmark) and were maintained in a temperature and light-controlled facility with free access to standard rodent chow and water. Mice were randomized to the respective groups five days before start of treatment and were kept 5 to 6 animals per cage. Following the acclimatization period, acute colitis was induced by administration of 5% DSS solution (DSS from TdB Consultary AB,Sweden; MW~40000) for five days (day 0 to day 5) via the drinking water, which resulted in reproducible colitis characterized by diarrhoea, rectal bleeding, reduced body weight, mucosal ulceration, crypt destruction and infiltration of leukocytes. The DSS intake was assessed daily. SASP (from Sigma-Aldrich, Sweden) was first dissolved in 0.2 M sodium hydroxide (NaOH) at a concentration higher than the required final concentration. Phosphate buffered saline (PBS) was added to the desired concentration and the pH was adjusted to 7.5-8.0 with NaOH or hydrochloric acid. SASP remained completely dissolved at this pH. Cpd A was dissolved in PBS to the desired concentration. All animal groups had free access to 5% DSS water solution for the entire test period. Treatments were administered from day 0 to day 5, either orally by gavage (SASP) or intraperitoneally (Cpd A), and in an administration volume of 5 ml/kg, twice daily with approximately an 8-hour interval. The control group (DSS only) received water (0.2 ml) orally twice daily.

The parameters recorded in the experiment were stool consistency (0, normal; 2, loose stools; 4, watery diarrhoea), occurrence of blood in the stool (0, normal; 2, Hemoccult+; 4, gross bleeding), and body weight loss (0, none; 1, 1-5%; 2, 5-10%; 3, 10-20%; 4, >20%). Disease activity index (DAI) was calculated by summarizing the scores of these three parameters. Also the colon length and the colonic content of myeloperoxidase (MPO) were recorded. Data was statistically evaluated with analysis of variance (ANOVA). Least-squares means (LS means), and standard error (SE) of LS means were calculated.

**Table 3. Percentage inhibition on day 5 of dextran sulphate sodium disease activity index (100% = complete normalisation, 0% = no effect) vs control.**

| **AABZ** | **Dose mg/kg/day bid ip** | **5-ASA Agent** | **Dose mg/kg/day bid po** | **Disease Activity Index** | | |
|---|---|---|---|---|---|---|
| | | | | LS means ±SE | p-value vs Control | Percent Reduction of Score |
| Control | | | | 6.9±0.3 | | |
| | | SASP | 40 | 4.4±0.4 | <0.0001 | 36 |
| Cpd A | 50 | | | 6.4±0.5 | 0.41 | 7 |
| Cpd A | 100 | | | 1.7±1.0 | <0.0001 | 75 |

**Table 4. Percentage inhibition on day 5 of dextran sulphate sodium blood in the stool score (100% = complete normalisation, 0% = no effect) vs control.**

| **AABZ** | **Dose mg/kg/day bid ip** | **5-ASA Agent** | **Dose mg/kg/day bid po** | **Blood in the Stool** | | |
|---|---|---|---|---|---|---|
| | | | | LS means ±SE | p-value vs Control | Percent Reduction of Score |
| Control | | | | 2.4±0.1 | | |
| | | SASP | 40 | 1.4±0.2 | <0.0001 | 42 |
| Cpd A | 50 | | | 2.2±0.3 | 0.45 | 9 |
| Cpd A | 100 | | | 0.1±0.5 | <0.0001 | 96 |

Administration of 5% DSS for 5 days resulted in severe acute colitis, with diarrhoea, blood in feces and weight loss. Diarrhoea and blood in feces appeared from day 3 and these symptoms aggravated until study termination on day 5. Oral treatment with the optimal dose (40 mg/kg/day bid) of SASP ameliorated these manifestations of colitis (Table 3). There was an improvement of diarrhoea as well as blood in feces from day 3 onwards. The frequency of diarrhoea (stool consistency score of 2 and 4) amounted to 22% in the control group and 8% in the SASP group on day 3. On day 5, the corresponding figures were 98% and 71%, respectively. The comparison of Cpd A 100 mg/kg/day bid to SASP 40 mg/kg/day bid treatment resulted in a superior amelioration of the colitis parameters in Cpd A treated animals. The Disease Activity Index (DAI) scores were significantly reduced by the Cpd A and SASP treatments by 75% and 36%, respectively (Table 3), and significantly reduced by Cpd A versus SASP treatment by 60% (p<0.016). There was a sharp reduction in the blood in feces score in the Cpd A 100 mg/kg/day bid group [(0.1±0.5 vs. 2.4±0.1 for the control group, p<0.0001) and (0.1±0.5 vs. 1.4±0.2 for the SASP group, p<0.024)] (Table 4)..

### EXAMPLE 7

### Receptor Affinity.

It is well-known that drug compounds that have an affinity for the striatal dopamine D2 may give rise to adverse side effects, such as short-term extrapyramidal disorders and tardive dyskinesia. Such adverse effects e.g. have been observed for metoclopramide, which reduces its utility in particular for the long-term treatment of chronic diseases. The in vitro affinity for the dopamine D2 receptor of Cpd A, and of two prior art compounds metoclopramide and declopramide was investigated using an in vitro radioligand binding technique with ¹²⁵I-spiperone as the radioactive ligand (Gundlach, 1984). As shown in Table 5, Cpd A displays a very low affinity for the dopamine D2 subtype receptor.

**Table 5. Dopamine D2 receptor affinity.**

| **Compound** | **IC50 (mM)** |
|---|---|
| Metoclopramide | 0.07 |
| Declopramide | 38.6 |
| Cpd A | 975 |

### REFERENCES

Frøslie KF et al., Gastroenterology. 2007 133:412-422.
Gundlach AL et al., Life Sciences 1984 35 1981-1988.
Harrington RA et al., Drugs 1983 25:451-94.
Harron DWG et al., Drugs 1990 39: 720-740.
Hindryckx P et al., J Crohns Colitis. 2018 12 105-119.
Ivanenkov YA et al., Mini Rev Med Chem. 2011 1:55-78.
Kim W et al., Mol Pharm. 2016 13:2126-35.
Lim WC et al., Cochrane Database Syst Rev. 2016 7:CD008870.
Peyrin-Biroulet L et al., Am J Gastroenterol. 2015 110:1324-38.
Perše M et al., J Biomed Biotechnol. 2012 2012:718617.
Sandborn WJ. Gastroenterol Hepatol (N Y). 2016 12:438-41.
Sands BE et al., Gastroenterology. 2014 147:618-627.
Vowinkel T et al., Dig Dis Sci. 2004 49 556-64.

## Claims

1. A compound of formula (I) 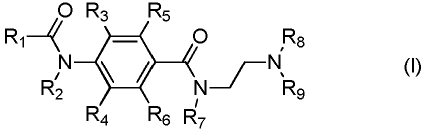
or a pharmaceutically acceptable salt or solvate thereof; wherein
R₁ is selected from C1-C6 alkyl and C3-C6 cycloalkyl;
R₂ is selected from H and C1-C3 alkyl;
R₃, R₄, R₅, and R₆ are independently selected from hydrogen, C1-C3 alkyl, C1-C3 alkoxy, C1-C3 alkylthio, fluoro, chloro, and bromo, wherein any alkyl is optionally substituted with one or more fluoro;
R₇ is selected from hydrogen and C1-C3 alkyl; and
R₈ and R₉ are independently selected from C1-C6 alkyl;
for use in the treatment of an inflammatory bowel disease.

2. The compound or pharmaceutically acceptable salt or solvate thereof for use according to claim 1, wherein
R₃, R₄, R₅, and R₆ are independently selected from hydrogen, C1-C2 alkyl, C1-C2 alkoxy, C1-C2 alkylthio, fluoro, chloro, bromo, and trifluoromethyl.

3. The compound or pharmaceutically acceptable salt or solvate thereof for use according to claim 1 or 2, wherein
R₁ is selected from C1-C3 alkyl;
each one of R₂, R₄, R₅, R₆ and R₇ is hydrogen; and
R₈ and R₉ are independently selected from C1-C4 alkyl.

4. The compound or pharmaceutically acceptable salt or solvate thereof for use according to any one of claims 1 to 3, wherein the compound is selected from
4-acetamido-3-chloro-N-[2-(diethylamino)ethyl]benzamide,
4-propanoylamino-3-chloro-N-[2-(diethylamino)ethyl]benzamide,
4-isobutyrylamino-3-chloro-N-[2-(diethylamino)ethyl]benzamide,
4-acetamido-N-[2-(diethylamino)ethyl]benzamide,
4-propanoylamino-N-[2-(diethylamino)ethyl]benzamide, and
4-isobutyrylamino-N-[2-(diethylamino)ethyl]benzamide.

5. The compound or pharmaceutically acceptable salt or solvate thereof for use according to claim 4, wherein the compound is 4-acetamido-3-chloro-N-[2-(diethylamino)ethyl]-benzamide.

6. The compound or pharmaceutically acceptable salt or solvate thereof for use according to claim 4, wherein the compound is 4-propanoylamino-3-chloro-N-[2-(diethylamino)ethyl]benzamide.

7. The compound or pharmaceutically acceptable salt or solvate thereof for use according to claim 4, wherein the compound is 4-isobutyrylamino-3-chloro-N-[2-(diethylamino)ethyl]benzamide.

8. The compound or pharmaceutically acceptable salt or solvate thereof for use according to claim 4, wherein the compound is 4-acetamido-N-[2-(diethylamino)ethyl]benzamide.

9. The compound or pharmaceutically acceptable salt or solvate for use according to any one of claims 1 to 8, in the treatment of a patient for which anti-TNF-α treatment has failed or is contraindicated.

10. A compound selected from
4-propanoylamino-3-chloro-N-[2-(diethylamino)ethyl]benzamide, and
4-isobutyrylamino-3-chloro-N-[2-(diethylamino)ethyl]benzamide, or
a pharmaceutically acceptable salt or solvate thereof.

11. The compound or pharmaceutically acceptable salt or solvate according to claim 10, for use in therapy.

12. The compound or pharmaceutically acceptable salt or solvate according to claim 10, for use in the treatment of an inflammatory bowel disease.

13. A pharmaceutical composition comprising the compound or pharmaceutically acceptable salt or solvate according to claim 10 and optionally a pharmaceutically acceptable excipient.

14. The compound or pharmaceutically acceptable salt or solvate for use according to any one of claims 1 to 9 or claim 12, wherein the inflammatory bowel disease is ulcerative colitis.

15. The compound or pharmaceutically acceptable salt or solvate for use according to any one of claims 1 to 9 or claim 12, wherein the inflammatory bowel disease is Crohn's disease.

## Patentansprüche

1. Verbindung der Formel (I) 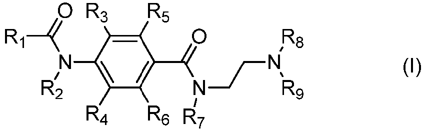
oder pharmazeutisch verträgliches Salz oder Solvat davon; wobei
R₁ aus C1-C6-Alkyl und C3-C6-Cycloalkyl ausgewählt ist;
R₂ aus H und C1-C3-Alkyl ausgewählt ist;
R₃, R₄, R₅ und R₆ unabhängig voneinander ausgewählt sind aus Wasserstoff, C1-C3-Alkyl, C1-C3-Alkoxy, C1-C3-Alkylthio, Fluor, Chlor, und Brom, wobei jedes Alkyl gegebenenfalls mit einem oder mehreren Fluoratomen substituiert ist;
R₇ aus Wasserstoff und C1-C3-Alkyl ausgewählt ist; und
R₈ und R₉ unabhängig voneinander ausgewählt sind aus C1-C6-Alkyl;
zur Verwendung bei der Behandlung einer entzündlichen Darmerkrankung.

2. Verbindung oder pharmazeutisch verträgliches Salz oder Solvat davon zur Verwendung gemäß Anspruch 1, wobei
R₃, R₄, R₅ und R₆ unabhängig voneinander ausgewählt sind aus Wasserstoff, C1-C2-Alkyl, C1-C2-Alkoxy, C1-C2-Alkylthio, Fluor, Chlor, Brom, und Trifluormethyl.

3. Verbindung oder pharmazeutisch verträgliches Salz oder Solvat davon zur Verwendung gemäß Anspruch 1 oder 2, wobei R₁ aus C1-C3-Alkyl ausgewählt ist;
jedes aus R₂, R₄, R₅, R₆ und R₇ Wasserstoff ist und
R₈ und R₉ unabhängig voneinander aus C1-C4-Alkyl ausgewählt sind.

4. Verbindung oder pharmazeutisch verträgliches Salz oder Solvat zur Verwendung gemäß einem der Ansprüche 1 bis 3, wobei die Verbindung ausgewählt ist aus
4-Acetamido-3-chlor-N-[2-(diethylamino)ethyl]benzamid,
4-Propanoylamino-3-chlor-N-[2-(diethylamino)ethyl]benzamid,
4-Isobutyrylamino-3-chlor-N-[2-(diethylamino)ethyl]benzamid,
4-Acetamido-N-[2-(diethylamino)ethyl]benzamid,
4-Propanoylamino-N-[2-(Diethylamino)ethyl]benzamid und
4-Isobutyrylamino-N-[2-(Diethylamino)ethyl]benzamid.

5. Verbindung oder pharmazeutisch verträgliches Salz oder Solvat davon zur Verwendung gemäß Anspruch 4, wobei die Verbindung ist 4-Acetamido-3-chlor-N-[2-(diethylamino)ethyl]benzamid.

6. Verbindung oder pharmazeutisch verträgliches Salz oder Solvat davon zur Verwendung gemäß Anspruch 4, wobei die Verbindung ist 4-Propanoylamino-3-chlor-N-[2-(diethylamino)ethyl]benzamid.

7. Verbindung oder pharmazeutisch verträgliches Salz oder Solvat davon zur Verwendung gemäß Anspruch 4, wobei die Verbindung ist 4-Isobutyrylamino-3-chlor-N-[2-(diethylamino)ethyl]benzamid.

8. Verbindung oder pharmazeutisch verträgliches Salz oder Solvat davon zur Verwendung gemäß Anspruch 4, wobei die Verbindung ist 4-Acetamido-N-[2-(diethylamino)ethyl]benzamid.

9. Verbindung oder pharmazeutisch verträgliches Salz oder Solvat zur Verwendung gemäß einem der Ansprüche 1 bis 8, bei der Behandlung eines Patienten, bei dem eine Anti-TNF-α-Behandlung fehlgeschlagen ist oder kontraindiziert ist.

10. Verbindung, ausgewählt aus
4-Propanoylamino-3-chlor-N-[2-(diethylamino)ethyl]benzamid und
4-Isobutyrylamino-3-chlor-N-[2-(diethylamino)ethyl]benzamid, oder
ein pharmazeutisch verträgliches Salz oder Solvat davon.

11. Verbindung oder pharmazeutisch verträgliches Salz oder Solvat gemäß Anspruch 10 zur Verwendung in der Therapie.

12. Verbindung oder pharmazeutisch verträgliches Salz oder Solvat gemäß Anspruch 10 zur Verwendung bei der Behandlung einer entzündlichen Darmerkrankung.

13. Pharmazeutische Zusammensetzung, die die Verbindung oder das pharmazeutisch verträgliche Salz oder Solvat gemäß Anspruch 10 und optional einen pharmazeutisch verträglichen Hilfsstoff umfasst.

14. Verbindung oder pharmazeutisch verträgliches Salz oder Solvat zur Verwendung gemäß einem der Ansprüche 1 bis 9 oder Anspruch 12, wobei die entzündliche Darmerkrankung Colitis ulcerosa ist.

15. Verbindung oder pharmazeutisch verträgliches Salz oder Solvatt zur Verwendung gemäß einem der Ansprüche 1 bis 9 oder Anspruch 12, wobei die entzündliche Darmerkrankung Morbus Crohn ist.

## Revendications

1. Composé de formule (I) 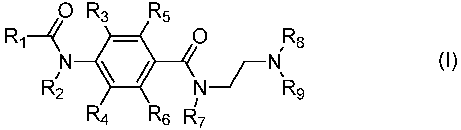
ou un sel ou solvate pharmaceutiquement acceptable de celui-ci ; dans lequel
R₁ est sélectionné parmi un alkyle en C₁ à C₆ et un cycloalkyle en C₃ à C₆ ;
R₂ est sélectionné parmi H et un alkyle en C₁ à C₃ ;
R₃, R₄, R₅ et R₆ sont sélectionnés indépendamment parmi un hydrogène, un alkyle en C₁ à C₃, un alcoxy en C₁ à C₃, un alkylthio en C₁ à C₃, fluoro, chloro, et bromo, dans lequel n'importe quel alkyle est facultativement substitué avec un ou plusieurs fluoros ;
R₇ est sélectionné parmi un hydrogène et un alkyle en C₁ à C₃ ; et
R₈ et R₉ sont sélectionnés indépendamment parmi un alkyle en C₁ à C₆ ;
pour une utilisation dans le traitement d'une une maladie inflammatoire de l'intestin.

2. Composé ou sel ou solvate pharmaceutiquement acceptable de celui-ci pour une utilisation selon la revendication 1, dans lequel
R₃, R₄, R₅ et R₆ sont sélectionnés indépendamment parmi un hydrogène, un alkyle en C₁ à C₂, un alcoxy en C₁ à C₂, un alkylthio en C₁ à C₂, fluoro, chloro, bromo, et un trifluorométhyle.

3. Composé ou sel ou solvate pharmaceutiquement acceptable de celui-ci pour une utilisation selon la revendication 1 ou 2, dans lequel
R₁ est sélectionné parmi un alkyle en C₁ à C₃ ;
chacun parmi R₂, R₄, R₅, R₆ et R₇ est un hydrogène ; et
R₈ et R₉ sont sélectionnés indépendamment parmi un alkyle en C₁ à C₄.

4. Composé ou sel ou solvate pharmaceutiquement acceptable de celui-ci pour une utilisation selon l'une quelconque des revendications 1 à 3, dans lequel le composé est sélectionné parmi
le 4-acétamido-3-chloro-N-[2-(diéthylamino)éthyl]benzamide,
le 4-propanoylamino-3-chloro-N-[2-(diéthylamino)éthyl]benzamide,
le 4-isobutyrylamino-3-chloro-N-[2-(diéthylamino)éthyl]benzamide,
le 4-acétamido-N-[2-(diéthylamino)éthyl]benzamide,
le 4-propanoylamino-N-[2-(diéthylamino)éthyl)benzamide, et
le 4-isobutyrylamino-N-[2-(diéthylamino)éthyl]benzamide.

5. Composé ou sel ou solvate pharmaceutiquement acceptable de celui-ci pour une utilisation selon la revendication 4, dans lequel le composé est le 4-acétamido-3-chloro-N-[2-(diéthylamino)éthyl]benzamide.

6. Composé ou sel ou solvate pharmaceutiquement acceptable de celui-ci pour une utilisation selon la revendication 4, dans lequel le composé est le 4-propanoylamino-3-chloro-N-[2-(diéthylamino)éthyl]benzamide.

7. Composé ou sel ou solvate pharmaceutiquement acceptable de celui-ci pour une utilisation selon la revendication 4, dans lequel le composé est le 4-isobutyrylamino-3-chloro-N-[2-(diéthylamino)éthyl]benzamide.

8. Composé ou sel ou solvate pharmaceutiquement acceptable de celui-ci pour une utilisation selon la revendication 4, dans lequel le composé est le 4-acétamido-N-[2-(diéthylamino)éthyl]benzamide.

9. Composé ou sel ou solvate pharmaceutiquement acceptable pour une utilisation selon l'une quelconque des revendications 1 à 8, dans le traitement d'un patient pour lequel le traitement anti-TNF-α a échoué ou est contre-indiqué.

10. Composé choisi parmi
le 4-propanoylamino-3-chloro-N-[2-(diéthylamino)éthyl]benzamide, et
le 4-isobutyrylamino-3-chloro-N-[2-(diéthylamino)éthyl]benzamide, ou
un sel ou solvate pharmaceutiquement acceptable de celui-ci.

11. Composé ou sel ou solvate pharmaceutiquement acceptable selon la revendication 10, pour une utilisation en thérapie.

12. Composé ou sel ou solvate pharmaceutiquement acceptable selon la revendication 10, pour une utilisation dans le traitement d'une une maladie inflammatoire de l'intestin.

13. Composition pharmaceutique comprenant le composé ou sel ou solvate pharmaceutiquement acceptable selon la revendication 10, et facultativement un excipient pharmaceutiquement acceptable.

14. Composé ou sel ou solvate pharmaceutiquement acceptable pour une utilisation selon l'une quelconque des revendications 1 à 9 ou la revendication 12, dans lequel la maladie inflammatoire de l'intestin est la colite ulcéreuse.

15. Composé ou sel ou solvate pharmaceutiquement acceptable pour une utilisation selon l'une quelconque des revendications 1 à 9 ou la revendication 12, dans lequel la maladie inflammatoire de l'intestin est la maladie de Crohn.
